# EUROPEAN PATENT SPECIFICATION

(11) **EP 2 028 209 B1**
(45) Date of publication and mention of the grant of the patent: **13.08.2014**
(21) Application number: 07745071.6
(22) Date of filing: 12.06.2007
(51) Int. Cl.: C08G 63/82, C07C 309/06, C07C 211/52, C07D 233/58, C07D 213/18

(54) **Method for producing poly-L-lactic acid**
Verfahren zur Herstellung von Poly-L-Milchsäure
Procédé de production d'acide poly-L-lactique

(30) Priority: 15.06.2006 JP 2006165991
(43) Date of publication of application: 25.02.2009
(73) Proprietor: National University Corporation Kyoto Institute of Technology, Sakyo-ku Kyoto-shi, Kyoto 606-8585 (JP)
(72) Inventor: ABIKO, Atsushi, Kyoto-shi, Kyoto 606-8585 (JP); IWAHASHI, Hisako, Kyoto-shi, Kyoto 606-8585 (JP)
(74) Representative: HOFFMANN EITLE
(86) International application number: PCT/JP2007/061784
(87) International publication number: WO 2007/145195

(56) References cited:
- EP-A1- 0 665 211
- JP-A- 05 105 745
- JP-A- 2002 053 649
- JP-A- 2002 249 953
- JP-A- 2003 105 073
- DATABASE WPI Week 200431 Thomson Scientific, London, GB; AN 2004-334005 XP002613962, -& JP 2004 043727 A (NISHIKAWA GOMU KOGYO KK) 12 February 2004 (2004-02-12)
- DATABASE WPI Week 200407 Thomson Scientific, London, GB; AN 2004-064950 XP002613963, -& JP 2003 105073 A (ASAHI KASEI KK) 9 April 2003 (2003-04-09)

## Description

### THECHNICAL FIELD

The present invention relates to an organic-acid-based catalyst for direct dehydration polycondensation of hydroxycarboxylic acids, in particular L-lactic acid.

### BACKGROUND ART

Polyhydroxycarboxylic acids such as poly-L-lactic acid, which are superior in mechanical, physical and chemical properties and biodegradable, i.e., decomposed under natural environment by microorganisms finally into water and carbon dioxide gas, are recently attracting attention in various fields, for example, as a medical material and a general-purpose resin substitute, and therefore there is expected large increase in its demands in the future.

Poly-L-lactic acid is prepared as described below by ring-opening polymerization of L-lactide, cyclic diester monomer of lactic acid (lactide method) or by indirect polymerization method, for example, via L-lactic acid oligomer ("lactide method" is also included in the indirect polymerization method).

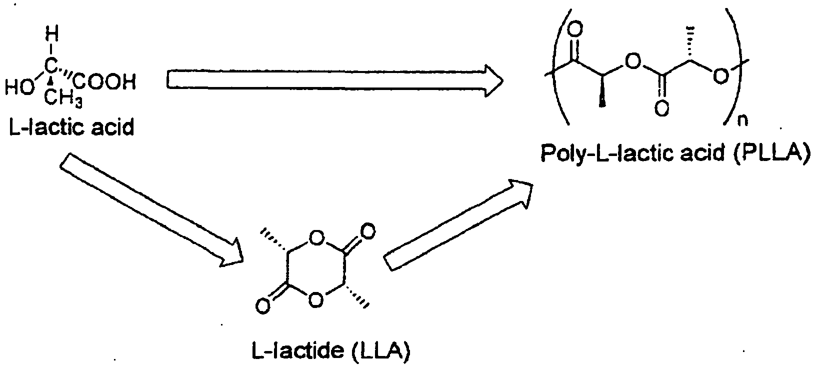

The lactide method gives high-molecular weight poly-L-lactic acids as the raw material is purified by isolation of lactide, but industrial lactide production and purification demanded significantly cost increase in operation and facility, causing a problem in producing inexpensive product.

There are only limited kinds of the catalysts used in the indirect polymerization method, and there are reports of using p-toluenesulfonic acid, tin chloride, or tin chloride and p-toluenesulfonic acid in combination as the catalyst (Nonpatent Documents 1 to 4).

In the report of using p-toluenesulfonic acid or tin chloride (Nonpatent Document 1), lactic acid is first converted to an oligomer at a catalyst/L-lactic acid ratio of 2.5 wt% and then polymerized by azeotropic dehydration polycondensation by using molecular sieve to a polymer having a molecular weight of about 100,000.

In the report of the combined use of tin chloride and p-toluenesulfonic acid as a catalyst (Nonpatent Document 2), lactic acid is first converted to an oligomer while heated in the absence of catalyst under reduced pressure and then melt-polymerized in the presence of an added catalyst (catalyst/oligo(L-lactic acid) ratio: 0.4 wt%) to a polymer having a molecular weight of about 20,000, crystallized under heat, and post-polymerized in solid phase to give a polymer having a molecular weight of about 100,000.

Although there are reports of a catalyst for direct polycondensation of lactic acid (Patent Documents 1-5), the methods have many problems in catalytic activity and process configuration such as low stability of the catalyst to water, low catalytic activity, necessity of complete removal of the catalyst because of use of metal catalyst etc., and for that reason, the indirect polymerization method has been used in practice.
Patent Document 1: Japanese Patent Application Laid-Open No. 2003-335850
Patent Document 2: Japanese Patent Application Laid-Open No. 10-231358
Patent Document 3: Japanese Patent Application Laid-Open No. 2001-213949
Patent Document 4: Japanese Patent Application Laid-Open No. 2002-138142
Patent Document 5: Japanese Patent Application Laid-Open No. 2004-43727
Nonpatent Document 1: Ajioka M, Enomoto E, Suzuki K and Yamaguchi A, Bull. Chem. Soc. Jpn 1995, 68, 2125.
Nonpatent Document 2: S.I. Moon, I. Taniguchi, M. Miyamato, Y. Kimura and C.W. Lee., High Perform. Polymer, 2001, 13, S189-S196.

### Disclosure of Invention

### Technical Problems to be Solved

An object of the present invention, which was made under the circumstances above, is to provide a new organic-acid-based catalyst that can synthesize poly-L-lactic acid by direct dehydration polycondensation of L-lactic acid.

### Means to Solve the Problems

Thus, the present invention provides an amine salt or phosphine salt of sulfonic acid as a new organic-acid-based catalyst able to synthesize poly-L-lactic acid.

### Advantageous Effect of the Invention

The organic-acid-based catalyst according to the present invention having a catalytic activity higher than those of conventional direct polymerization catalysts allows direct production of polylactic acid from lactic acid. Thus, use of it leads to simplification of the production process and improvement in productivity.

In addition, the organic-acid-based catalyst according to the present invention can be reused. Thus, use of it also leads to cost reduction and also waste reduction.

### Brief Description of Drawings

Figure 1 is a graph showing relationship between catalyst concentration and molecular weight.
Figure 2 is a ¹H-NMR spectrum of a poly-L-lactic acid.
Figure 3 is a GPC chromatogram.
Figure 4 is a ¹H-NMR spectrum of a poly-L-lactic acid.
Figure 5 is a ¹H-NMR spectrum of a L-lactic acid oligomer.

### Best Mode for Carrying Out the Invention

The organic-acid-based catalyst according to the present invention is an amine salt of sulfonic acid or a phosphine salt of sulfonic acid.

The word of sulfonic acid in the present invention is used as a concept to include alkanesulfonic and arenesulfonic acids. An alkanesulfonic acid is used favorably. Those sulfonic acids are preferably substituted with fluorine atoms, and the number of substitution is preferably as large as possible. In particular, use of a fluorine-substituted alkanesulfonic acid such as trifluoromethanesulfonic acid is the most preferable.

The word of amine in the present invention is used as a concept to include aliphatic and/or aromatic primary, secondary, and tertiary amines, polyamines such as diamines and triamines etc. containing those amines, and nitrogen-containing heterocyclic compounds (monocyclic and fused rings). The amine forming a salt with sulfonic acid is an alkylamine, an aromatic amine, or a nitrogen-containing heterocyclic compound, preferably an aromatic amine or a nitrogen-containing heterocyclic compound. Examples of the alkylamine include triethylamine, diethylcyclohexylamine, and the like; examples of the aromatic amine include aniline, diphenylamine and the like; and examples of the nitrogen-containing heterocyclic compound include pyridine, N-methylimidazole, and the like. Among those amines above, fluorine-substituted amines, in particular, aromatic amines, are preferable, and the substitution number thereof is favorably as large as possible. The fluorine-substituted aromatic amine is, for example, 2,3,4,5,6-pentafluoroaniline.

Examples of the phosphine forming a salt with sulfonic acid include monophosphines such as triarylphosphines, aryldialkylphosphines, diarylalkylphosphines and trialkylphosphines; diphosphines, triphosphines and others in combination thereof; and the like. An aryl group-containing phosphine is preferable, and an example of such phosphines is triphenylphosphine.

The amine salt or phosphine salt of a sulfonic acid may be prepared by any known methods, but is generally prepared by adding an equivalent amount of a sulfonic acid to an amine or phosphine dissolved in an inert solvent such as methylene chloride dropwise while the mixture is cooled on ice, precipitating the salt by dilution with a salt-incompatible solvent such as ether, and collecting the precipitate by filtration.

The combination of the sulfonic acid and the amine or phosphine is adjusted so that the organic-acid-based catalyst according to the present invention has an acid dissociation constant (K) as high as possible. Production of polylactic acid from lactic acid, which is fundamentally an esterification reaction, is carried out by azeotropic dehydration in the presence of an acid catalyst. The organic-acid-based catalyst according to the present invention, which provides proton ions (H⁺) then as an acid catalyst, is lower in catalytic activity when its dissociation constant is excessively low.

L-lactic acid is normally available in a water-containing form. Poly-L-lactic acid is prepared by dehydration condensation of such a lactic acid as above and a catalyst according to the present invention in a suitable solvent. The catalyst according to the present invention is stable to water and can synthesize directly poly-L-lactic acid from L-lactic acid without through a step of forming a dimer or oligomer.

The concentration of the catalyst to lactic acid (catalyst/lactic acid) is normally in the range of 0.01 to 1 mol %. The organic-acid-based catalyst according to the present invention has sufficiently high activity especially at a lower concentration of about 0.1 mol %.

The solvent is used for azeotropic removal of water generated in the dehydration condensation reaction. Examples of the solvents for use include benzene, toluene, xylene and the like. The amount is preferably about 1 to 3 times (by volume) larger than that of lactic acid, from the viewpoint of operational convenience.

The reaction temperature is preferably higher, but is the azeotropic temperature of the solvent, because azeotropic dehydration is needed. Thus from the viewpoint of reaction temperature, benzene, toluene, and xylene, which have an azeotropic temperature higher in that order, are more preferable in that order.

The maximum molecular weight of the polymer prepared seems to be dependent on the reaction temperature, and the polymerization is continued for the period until the molecular weight reaches the maximum value at the temperature. Thus, the polymerization period is a condition to be determined properly according to desirable molecular weight, polymerization temperature, kind of catalyst and concentration of catalyst etc.

The catalyst according to the present invention can produces a poly-L-lactic acid having a molecular weight (Mw) of approximately 10000 to 100000 without racemization of L-lactic acid by polymerization.

The organic-acid-based catalyst according to the present invention has been described by taking poly-L-lactic acid as an example, but can also be used in production of polyhydroxycarboxylic acids other than poly-L-lactic acid, such as those from D-lactic acid, DL-lactic acid, glycolic acid, mandelic acid, 2-hydroxyiso-valeric acid, 2-hydroxybutanoic acid, malic acid, tartaric acid, and amino acids, such as L-phenylalanine, glycine, and L-α-alanine; and production of copolymers thereof. The organic-acid-based catalyst according to the present invention is also usable when the polyhydroxycarboxylic acid is partially replaced with a polyvalent alcohol such as 1,4-butanediol, in particular, bivalent alcohol and/or a polyvalent carboxylic acid such as succinic acid, in particular, bivalent carboxylic acid or a lactone.

Particularly when a copolymer of a hydroxycarboxylic acid such as L-lactic acid and an amino acid above is produced by using the organic-acid-based catalyst according to the present invention, it is possible to prepare a copolymer having a molecular weight in the order of thousands to tens of thousands, by properly adjusting the conditions including polymerization temperature, use of oligo lactic acid and polymerization method such as bulk polymerization.

The organic-acid-based catalyst according to the present invention can be reused. It is possible to recover the catalyst after polymerization, by diluting the reaction mixture with methanol, filtrating and separating the polymer obtained after precipitation, and removing the solvent from the filtrate. After removal of the solvent from the filtrate, the recovered catalyst may be used in the following reaction as it is or as needed after purification by recrystallization.

### Examples

### Analytical Instruments

### Measurement of ¹H-NMR (500 MHz) spectrum:

BRUKERDRX 500 spectrometer (manufactured by Bruker) was used.

The solvent used was CDCl₃ containing tetramethylsilane (TMS) as a standard compound in an amount of 0.03 vol %.

Measurement of weight-average molecular weight (Mw) and molecular weight distribution (Mw/Mn) of polymer Measurement was made by gel-permeation chromatography (GPC). Used in measurement were Shimadzu LC-6 AD pump, RID-10A RI detector, Shimadzu CLASS-LC10 Chromatopac data processor, and Shimadzu DGU-20A3 degasser. Columns used were TSK-GEL G1000H, G2000H and G2500H, and tetrahydrofuran (THF) was run at an elution flow rate of 1.0 ml/min at an oven temperature of 40°C. Measurement was performed by injecting 5 µl of the sample prepared by dissolving 40.0 mg of a polymer in 1.0 ml of THF. The weight-average molecular weight (Mw) and the molecular weight distribution (Mw/Mn) were calibrated by using polystyrene standard samples.

### Reagents used

90 wt% aqueous L-lactic acid solution, SnCl₂, and p-toluenesulfonic acid hydrate, each of which is a product of Wako Pure Chemical Industries, Ltd.
Trifluoromethanesulfonic acid: product of TOKYO CHEMICAL INDUSTRY CO., LTD
Pyridine: product of TOKYO CHEMICAL INDUSTRY CO., LTD
N-Methylimidazole: product of TOKYO CHEMICAL INDUSTRY CO., LTD
Triphenylphosphine: product of Wako Pure Chemical Industries, Ltd

### Preparation of 2,3,4,5,6-pentafluorophenylammonium triflate (PFPAT)

5.0 g of 2,3,4,5,6-pentafluoroaniline was dissolved in 25 ml of dichloromethane, 2.4 ml of CF₃SO₃H was added gradually dropwise to the solution and agitated while being cooled on ice. The precipitated crystal was collected by suction-filtration, washed with diethylether and dried under reduced pressure, to give a slightly-purple milky white crystal. The yield was 79.6%. The melting point thereof was 211.5°C.

### Preparation of pyridinium triflate

0.82 ml of pyridine was dissolved in 25 ml of methylene chloride in a 200-ml round-bottomed flask, and 0.9 ml of trifluoromethanesulfonic acid was added gradually dropwise while the mixture was agitated and cooled on ice. The precipitated crystal was suction-filtered and dried under reduced pressure. The yield was 94.6%.
Reference document: R. Cordone, W. D. Harman and H. Taube, J. Am. Chem. Soc. 1989, 111, 2896-2900.

### Preparation of N-methylimidazolium triflate

0.81 ml of N-methylimidazole was dissolved in 25 ml of methylene chloride in a 200-ml round-bottomed flask, and 0.9 ml of trifluoromethanesulfonic acid was added gradually dropwise while the mixture was agitated and cooled on ice. The precipitated crystal was suction-filtered and dried under reduced pressure. The yield was 92.3%.
Reference document: Chaoyu Xie, M. A. Staszak, J. T. Quatroche, C. D. Sturgill, V. V. Khau, and M.J. Martinelli, Organic Process Research & Development.2005, 9(6), 730-737.

### Preparation of triphenylphosphonium triflate TPPT

2.3 g of triphenylphosphine was dissolved in 25 ml of methylene chloride in a 200-ml round-bottomed flask, and 0.9 ml of trifluoromethanesulfonic acid was added gradually dropwise while the mixture was agitated and cooled on ice. The crystal obtained by precipitation from a solvent of methylene chloride/diethylether/hexane at 2/2/1 was suction-filtered and dried under reduced pressure. The yield was 78.9%
Reference document: van der Akker, and M. Jellinek, Recl. Trav. Chim. Pays-Bas, 1967, 86, 275-288.

### (Example 1)

4.0 ml of 90% aqueous L-lactic acid solution, 8.0 ml of a solvent (toluene), 25 ml of the catalyst shown in the following Table 1 (catalyst/L-lactic acid ratio: 1.00 mol%) were mixed in a flask. The mixture was azeotropically dehydrated under atmospheric pressure for 22.5 hours while a Dean Stark trap was installed and water was distilled off from the system.

Toluene was distilled (evaporated) from the polymer solution obtained. The residue was dissolved in 20 ml of dichloromethane (CH₂Cl₂), and the solution obtained was poured into 150 ml of ice-cooled methanol (CH₃OH) for precipitation of the polymer. The precipitate was suction-filtered and dried under reduced pressure. Polymer particles having a diameter allowing permeation through the filter paper were separated from methanol by centrifugation and dissolved in 10 ml of dichloromethane; the solvent was distilled (evaporated) from the solution obtained; and the residue was dried under reduced pressure, to give a polymer. Also in the following preparation of poly-L-lactic acid, a poly-L-lactic acid was prepared in a manner similar to that above, except that solvent, reaction period, catalyst and catalyst/L-lactic acid ratio were modified.

**[Table 1]**

| | Mw(GPC) (NMR) | Mw/Mn (GPC) | Yield (%) (%) |
|---|---|---|---|
| Without catalyst | 360 | | |
| SnCl₂ | 7900 | 1.40 | 63.3 |
| p-Toluenesulfonic acid | 23000 | 1.62 | |
| 2,3,4,5,6-Pentafluorophenylammonium triflate (PFPAT) | 19000 | 1.25 | 44.8 |
| Pyridinium triflate | 13100 | 1.34 | 73.8 |
| N-Methylimidazolium triflate | 8700 | 1.19 | 32.9 |
| Triphenylphosphonium triflate | 8000 | 1.11 | 21.6 |

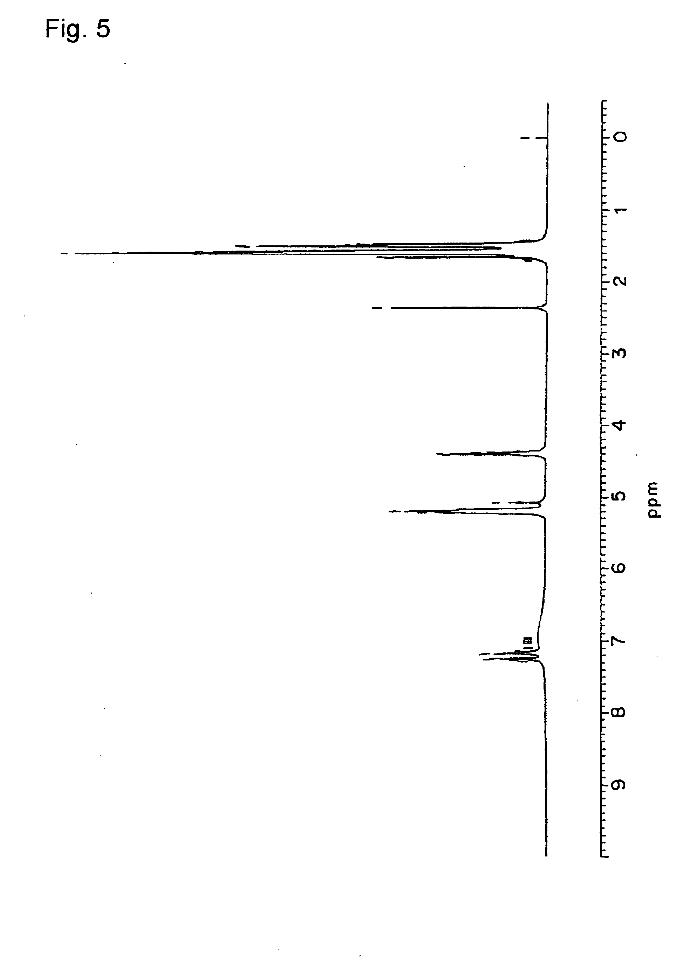

An oligomer was generated when the reaction was carried out without any catalyst. The ¹H NMR spectrum of the oligomer is shown in Figure 5. When a commonly used acid catalyst such as SnCl₂ or p-toluenesulfonic acid is used, the molecular weight of poly-L-lactic acid prepared under the conditions above was 7900 and 23000 in weight average molecular weight.

When one of the similar tin-based catalysts, such as di-n-butyltin diacetate, di-n-butyltin oxide, Sn(II)2-ethylhexanoate or Sn(III) trifluoromethanesulfonate, or alternatively pyridinium p-toluenesulfonate is used, there was no polymer generated (molecular weight: up to approximately 500).

In contrast, combined use of a salt of trifluoromethanesulfonic acid and pentafluoroaniline, PFPAT, gave a poly-L-lactic acid having a molecular weight of 18600.

Only an oligomer was formed under the condition where pyridinium p-toluenesulfonate was used, seemingly because p-toluenesulfonic acid, which has an acidity lower than that of trifluoromethanesulfonic acid, has a decreased acidity when bound to a weak base, forming a salt.

### (Example 2)

Subsequently, the catalytic activities of favorable catalysts, PFPAT, pyridinium triflate, N-methylimidazolium triflate, triphenylphosphonium triflate, p-toluenesulfonic acid and SnCl₂, were compared. Azeotropic dehydration was carried out in toluene for specific time shown in Table 2, and the relationship between reaction period and molecular weight was evaluated. Results are summarized in Table 2.

**[Table 2]**

| | Catalyst/LA (mol%) | Period (h) | Mw(GPC) (NMR) | Mw/Mn (GPC) | Yield (%) |
|---|---|---|---|---|---|
| SnCl₂ | 1.00×10⁻¹ | 22.5 | 360 | | |
| | 1.00×10⁻¹ | 72 | 9700 | 1.02 | 43.8 |
| p-Toluenesulfonic acid | 1.00 | 22.5 | 23000 | 1.62 | 65.1 |
| | 1.00 | 72 | 20000 | 1.53 | 77.2 |
| | 1.00×10⁻¹ | 22.5 | 4000 | 1.71 | 45.3 |
| | 1.00×10⁻¹ | 72 | 11000 | 1.74 | 75.1 |
| PFPAT | 1.00 | 22.5 | 19000 | 1.25 | 44.8 |
| | 1.00 | 72 | 18000 | 1.66 | 43.0 |
| | 1.00×10⁻¹ | 22.5 | 48000 | 1.73 | 97.7 |
| | 1.00×10⁻¹ | 72 | 52000 | 1.63 | 97.0 |
| Pyridinium triflate | 1.00 | 22.5 | 13000 | 1.34 | 73.8 |
| | 1.00×10⁻¹ | 72 | 8200 | 1.81 | 55.4 |
| N-Methylimidazolium triflate | 1.00 | 22.5 | 8700 | 1.19 | 32.9 |
| | 1.00×10⁻¹ | 72 | 3400 | 1.44 | 28.0 |
| Triphenylphosphonium triflate | 1.00 | 22.5 | 8000 | 1.11 | 21.6 |
| | 1.00×10⁻¹ | 22.5 | 11000 | 1.26 | 88.5 |
| | 1.00×10⁻¹ | 72 | 18000 | 1.38 | 82.6 |

SnCl₂ gave an oligomer under the conditions of a catalyst/L-lactic acid ratio of 1.00x10⁻¹ mol % and a reaction period of 22.5 hours. Elongation of the reaction period to 72 hours resulted in production of a polymer having a molecular weight of 9700.

Elongation of the reaction period with p-toluenesulfonic acid leads to increase in molecular weight to some extent, but, if the catalyst amount was reduced to 1.00x10⁻¹ mol %, elongation of the reaction period did not lead to increase in molecular weight to that obtained when the catalyst amount was 1.00 mol %. It is probably due to inactivation of the catalyst caused by esterification of p-toluenesulfonic acid.

When PFPAT was used, it was possible to reduce the catalyst amount, compared to the other catalysts above. Polylactic acid having a molecular weight of about 18000 was obtained at a catalyst concentration of 1.0 mol %; the molecular weight thereof when the catalyst amount was reduced to 1.00×10⁻¹ mol % was 48000 after reaction for 22.5 hours and 52000 after reaction for 72 hours, indicating that the lower catalyst concentration was effective in giving a high-molecular weight under the same condition.

### (Example 3)

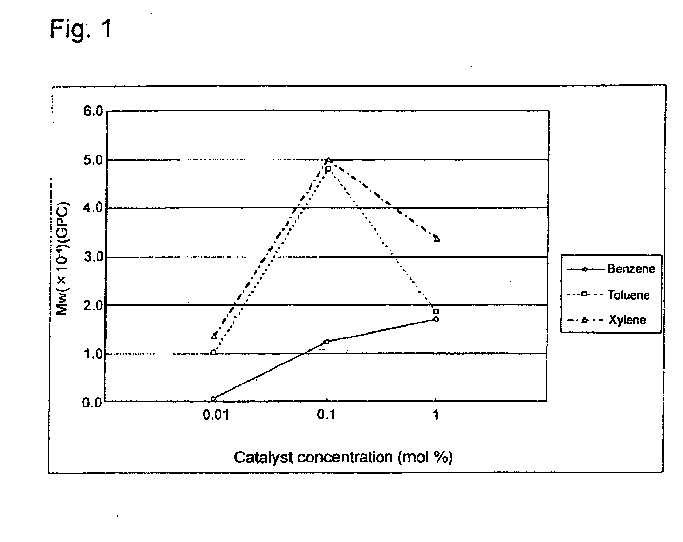

The reaction conditions with PFPAT were optimized, while the catalyst concentration and the solvent (the reaction temperature being the boiling point of each solvent) were altered, as shown in Table 3. The reaction period was 22.5 hours. Results are summarized in Table 3. The results in Table 3 are plotted in the graph of Figure 1.

**[Table 3]**

| Solvent | Catalyst/LA (mol%) | Mw(GPC) (NMR) |
|---|---|---|
| Benzene | 1.00×10⁻² | 450 |
| | 1.00×10⁻¹ | 12000 |
| | 1.00 | 17000 |
| Toluene | 1.00×10⁻² | 10000 |
| | 1.00×10⁻¹ | 48000 |
| | 1.00 | 19000 |
| Xylene | 1.00×10⁻² | 14000 |
| | 1.00×10⁻¹ | 50000 |
| | 1.00 | 34000 |

When the catalyst concentration was 1.00 mol %, the reaction in benzene gave a polymer having a molecular weight of 17000, the reaction in toluene, a molecular weight of 19000; and the reaction in xylene, a molecular weight of 34000.

When the catalyst concentration was 1.00×10⁻¹ mol %, the reaction in toluene or xylene gave a poly-L-lactic acid having a molecular weight of around 50000. However, decrease in the catalyst concentration to 1.00 × 10⁻² mol % leads to decrease in molecular weight of the generated polymer to a value lower than that when the catalyst concentration was 1.00×10⁻¹ mol %.

The catalyst concentration is considered favorable in the range of 1.00×10⁻² to 1.0 mol %.

Raised reaction temperature leads to increase in molecular weight of the polymer prepared at any catalyst concentration.

### (Example 4)

The reaction was carried out with PFPAT, pyridinium triflate, or triphenylphosphonium triflate, while catalyst concentration, reaction period and solvent were altered. Results are summarized in the following Table 4.

**[Table 4]**

| Reaction period and molecular weight | | | | | |
|---|---|---|---|---|---|
| | Catalyst/LA (mol%) | Solvent | Period (h) | Mw (GPC) | Mw/Mn (GPC) |
| Pyridinium triflate | 1 | Toluene | 5 | 6570 | 1.09 |
| | 1 | Toluene | 22.5 | 13100 | 1.34 |
| Triphenylphosphonium triflate | 0.1 | Xylene | 72 | 83400 | 1.71 |
| | 1 | Toluene | 5 | 4780 | 1.17 |
| | 1 | Toluene | 22.5 | 7970 | 1.11 |
| PFPAT | 0.1 | Xylene | 22.5 | 50100 | 1.49 |
| | 0.1 | Xylene | 72 | 107000 | 1.63 |
| | 1 | Xylene | 22.5 | 33700 | 1.44 |
| | 1 | Toluene | 3.5 | 10433 | 1.76 |
| | 1 | Toluene | 22.5 | 18600 | 1.25 |
| | 1 | Toluene | 72 | 17700 | 1.66 |
| | 1 | Benzene | 72 | 15800 | 1.50 |

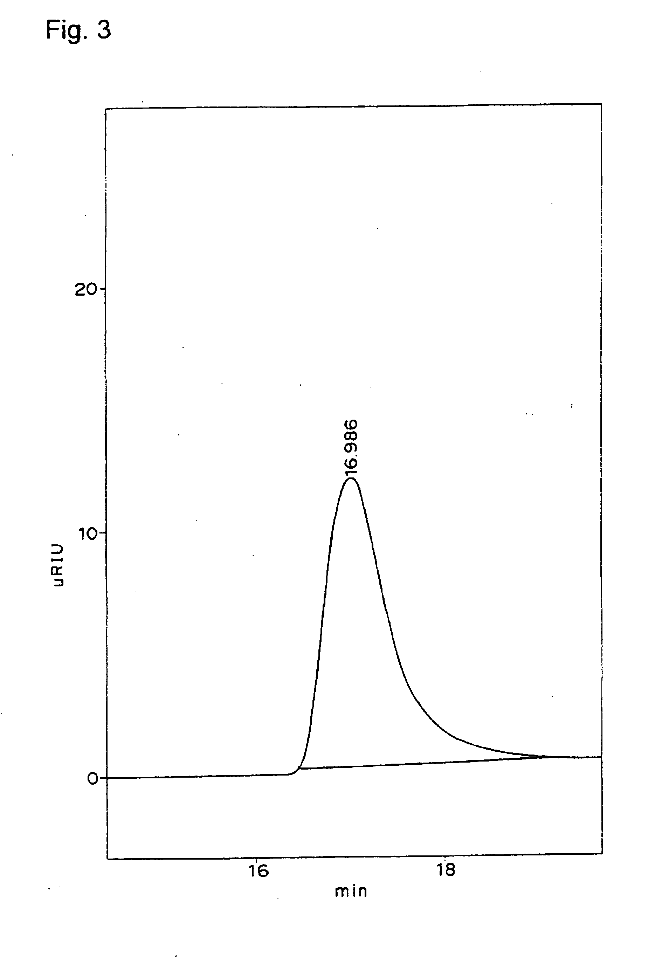

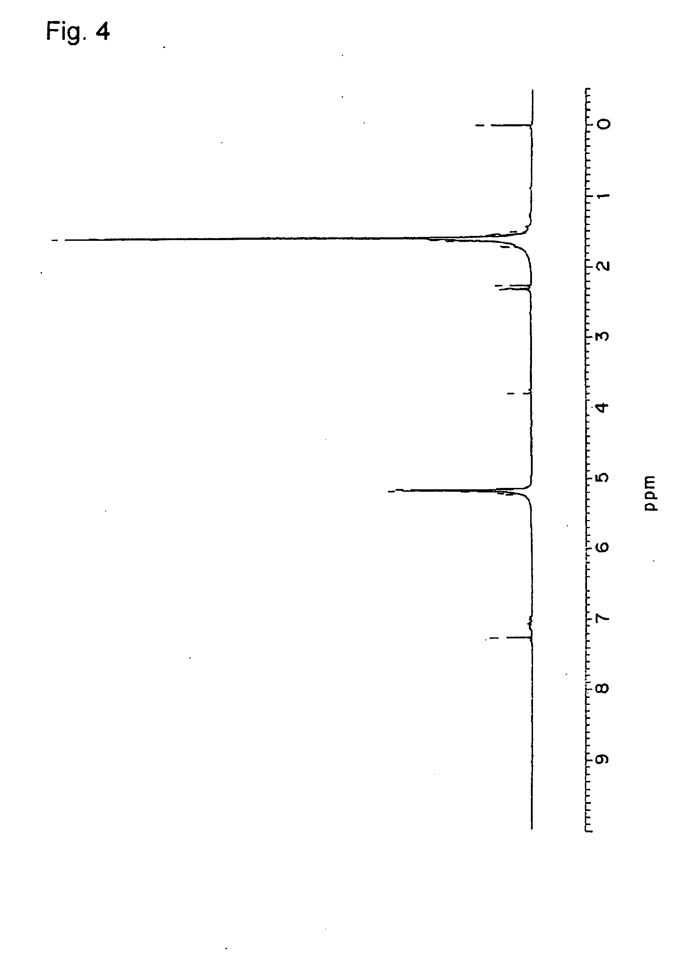

Elongation of the reaction period to 72 hours in xylene with PFPAT leads to increase in molecular weight to approximately 110000. The GPC chromatograph of a poly-L-lactic acid (reaction period: 22.5 hours, catalyst concentration (PFPAT/L-lactic acid ratio): 1.0 mol %, solvent: xylene) is shown in Figure 3. The ¹H NMR spectrum of a poly-L-lactic acid (reaction period: 72 hours, catalyst concentration (PFPAT/L-lactic acid ratio): 0.1 mol %, solvent: xylene) is shown in Figure 4.

In the case of triphenylphosphonium triflate, reaction in xylene at a catalyst amount of 1.00×10⁻¹ mol % for 72 hours gives a polylactic acid having a molecular weight of 83000. These results suggest that the molecular weight of the polymer prepared has an upper limit dependent on the reaction temperature and is not dependent on the period at a temperature higher than that.

### (Example 5)

Subsequently, the stereochemistry of the poly-L-lactic acid generated was evaluated. The ¹H-NMR of the decoupled secondary methyl group in a poly-L-lactic acid sample prepared in xylene by using PFPAT (reaction period: 22.5 hours, catalyst/L-lactic acid ratio: 0.1 mol %) was measured.

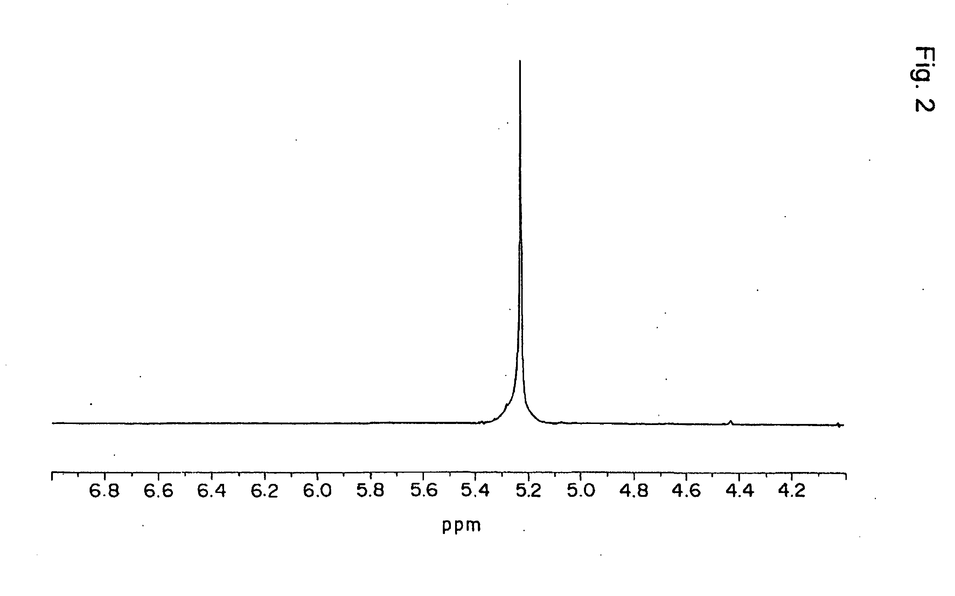

Results are shown in Figure 2. The spectrum of an almost single methine proton indicates that there was no racemization occurring in the reaction.

### (Example 6)

### Reuse of catalyst

Four ml of L-lactic acid, 8 ml of toluene and 10.7 mg of triphenylphosphonium triflate were mixed, and the mixture was subjected to polymerization reaction under atmospheric pressure for 22.5 hours while an Dean Stark trap was installed and water was distilled off from the system. After evaporation of toluene, the residue was dissolved in 20 ml of methylene chloride, and the resulting solution was poured into 150 ml of methanol for precipitation of the polymer. The precipitate was suction-filtered and dried under reduced pressure, and the mother liquid was recovered and evaporated. The concentrated mother liquid was mixed with 4 ml of L-lactic acid and 8 ml of toluene, and the mixture was subjected to polymerization reaction similarly, to give a polymer having a weight-average molecular weight of 7400 at a yield of 64.6%.

### Preparation of lactic acid-amino acid copolymer

L-lactic acid, an amino acid (L-phenylalanine, glycine or L-α-alanine) (10 mol % with respect to L-lactate), and the catalyst shown in the following Table 5 in an amount corresponding to a catalyst concentration of 0.1 mol % were mixed and subjected to azeotropic dehydration in toluene, allowing polymerization in a manner similar to Example 1 for 22.5 hours. The copolymer was isolated after polymerization by dissolving the polymer generated in CH₂ Cl₂ and reprecipitating it by addition of an excess amount of methanol. The precipitate obtained was suction-filtered and then dried under reduced pressure. Results are summarized in Table 5.

**[Table 5]**

| Amino acid | Catalyst | Mw | Mw/Mn | Yield (%) | Monomer composition (mol%)(by NMR) |
|---|---|---|---|---|---|
| | | | | | LA/comonomer |
| L-Phenylalanine | 2,3,4,5,6-Pentafluorophenylammonium triflate | 1300 | 1.20 | 46.6 | 83/17 |
| Glycine | Triphenylphosphonium triflate | 2000 | 2.17 | 86.0 | 96/4 |
| L-α-Alanine | Triphenylphosphonium triflate | 2000 | 1.14 | 77.5 | 93/7 |

The following polymerization method was evaluated for improvement in molecular weight of the copolymer.

### (1) Bulk polymerization

A mixture of L-lactic acid, L-phenylalanine (10 mol %), and a catalyst (PFPAT) (catalyst/monomer ratio: 1.00×10⁻¹ mol %) was oligomerized in toluene for 1 hour by azeotropic dehydration. The solvent was distilled from the solution, and the residue was heated under reduced pressure (20 mm Hg) at 170°C for 3 hours. The residue was further heated under reduced pressure (0.1 mm Hg) at 170°C for 2 hours. The copolymer was isolated in a manner similar to the preparation of lactic acid-amino acid copolymer above, to give a copolymer having a Mw of 5000, a Mw/Mm ratio of 1.31 and a monomer composition (LA/Phe) of 96/4 at a yield of 27.8%.

### (2) Reaction temperature (xylene solvent)

A mixture of L-lactic acid, L-phenylalanine (10 mol %), and a catalyst (PFPAT) (catalyst/monomer ratio: 1.00×10⁻¹ mol %) was subjected to azeotropic dehydration in xylene for 24 hours. The copolymer was isolated in a manner similar to the preparation of lactic acid-amino acid copolymer above, to give a copolymer having Mw of 9000 and Mw/Mm of 3.34 at a yield of 27.8%.

### (3) Use of oligo-lactic acid

A mixture of L-lactic acid and a catalyst (PFPAT) (catalyst/monomer ratio: 1.00×10⁻¹ mol %) was oligomerized in xylene for 24 hours by azeotropic dehydration. Then, L-phenylalanine was added in an amount of 10 mol % with respect to L-lactic acid, and the mixture was heated additionally for 22.5 hours. The copolymer was isolated in a manner similar to the preparation of the lactic acid-amino acid copolymer, to give a copolymer having Mw of 37200, Mw/Mn of 1.75 and monomer composition (LA/Phe) of 93/7 at a yield of 77.5%.

A mixture of 1,4-butanediol (BD) (100 mol %), succinic acid (SA) (100 mol %) and L-phenylalanine (10 mol %) was subjected to azeotropic dehydration in toluene with triphenylphosphonium triflate (catalyst/monomer ratio: 1.00×10⁻¹ mol %) for 22.5 hours. The copolymer was isolated in a manner similar to the preparation of lactic acid-amino acid copolymer above, to give a copolymer having Mw of 2600, Mw/Mn of 1.89 and a composition ratio PBS/L-Phe of 90/10 at a yield of 28.7%.

### Preparation of lactic acid-hydroxycarboxylic acid copolymer

A mixture of L-lactic acid (LA), glycolic acid (GA) or R-mandelic acid (MA) at the molar ratio shown in the following Table 6 was subjected to azeotropic dehydration in toluene solvent in the presence of triphenylphosphonium triflate (TPPT) (0.1 mol %) for 22 hours. The mixture was then cooled; toluene was evaporated; the residue was dissolved in methylene chloride and precipitated by addition of a large excess of methanol; and the precipitate was collected by filtration, to give a copolymer.

**[Table 6]**

| Mixed monomer composition (LA/GA) | Monomer composition (LA/GA) | Yield (%) | Mw | Mw/Mn |
|---|---|---|---|---|
| 100/10 | 100/10 | 61.3 | 36000 | 1.8 |
| 100/30 | 100/25 | 66.7 | 39000 | 1.5 |
| 100/70 | 100/77 | 60.8 | 41000 | 1.8 |
| 100/100 | 86/100 | 80.0 | 43000 | 2.0 |
| 70/100 | 63/100 | 69.4 | 46000 | 1.3 |
| 30/100 | 12/100 | 91.5 | * | |
| 10/100 | 10/100 | 89.4 | * | |
| LA/MA=100/10 | 100/5.5 | 34.2 | 31000 | 1.4 |

| | | | | |
|---|---|---|---|---|
| *GPC not performed because the copolymer was insoluble in THF | | | | |

### (Preparation of glycolic acid-hydroxy acid copolymer)

A mixture of glycolic acid (57 mol %) and DL-leucic acid (43 mol %) was allowed to react in the presence of a catalyst (TPPT 0.1 mol %) in toluene at 120°C for 24 hours. The yield was 12%. The copolymerization ratio, glycolic acid: leucic acid, of the copolymer obtained was 81:19 (molar ratio).

### (Preparation of glycolic acid-lactone copolymer)

A mixture of glycolic acid (57 mol %) and a lactone (43 mol %) was allowed to react in the presence of a catalyst (TPPT: 0.1 mol %) in xylene at 160°C for 24 hours, to give a copolymer. The lactone used was ε-caprolactone, δ-valerolactone, or γ-butyrolactone.

When ε-caprolactone was used, the yield was 74%, and the copolymerization ratio of the copolymer obtained, glycolic acid: caprolactone, was 50: 50 (by molar ratio).

When δ-valerolactone was used, the yield was 45%, and the copolymerization ratio, glycolic acid: valerolactone, of the copolymer obtained was 56:44 (by molar ratio).

When γ-butyrolactone was used, the yield was 61%, and the copolymerization ratio, glycolic acid: valerolactone, of the copolymer obtained was 87.5:12.5 (by molar ratio).

### (Preparation of succinic acid-diol-glycolic acid copolymer)

A mixture of succinic acid, a diol and glycolic acid at a molar ratio of 1.5:1.5:7 was allowed to react in the presence of catalyst (TPPT 0.1 mol %) in xylene at 160°C for 24 hours, to give a copolymer.

When the diol used was 1,4-butanediol, the yield was 47%, and the copolymerization ratio of the copolymer obtained, succinic acid: butanediol: glycolic acid, was 1:1:5 (by molar ratio).

When the diol used was ethylene glycol, the yield was 64%, and the copolymerization ratio of the copolymer obtained, succinic acid: ethylene glycol: glycolic acid, was 1:1:2 (by molar ratio).

### (Preparation of lactic acid-malic acid (monohydroxydicarboxylic acid) copolymer)

A mixture of lactic acid and malic acid at a ratio of 90:10 (mol %) was allowed to react in the presence of a catalyst (TPPT 0.1 mol %) in toluene at 130°C for 24 hours, to give a copolymer. The yield was 31%, and the copolymerization ratio of the copolymer obtained, lactic acid: malic acid, was 90:10 (by molar ratio).

## Claims

1. A method of producing poly-L-lactic acid, comprising using an organic-acid-based catalyst containing an amine salt of sulfonic acid or a phosphine salt of sulfonic acid in production of the poly-L-lactic acid by dehydration polycondensation of L-lactic acid.

2. A method of producing a polyhydroxycarboxylic acid, comprising using an organic-acid-based catalyst containing an amine salt of sulfonic acid or a phosphine salt of sulfonic acid in production of the polyhydroxycarboxylic acid by dehydration polycondensation of a hydroxycacrboxylic acid.

3. A method of producing a polyhydroxycarboxylic acid copolymer, comprising using an organic-acid-based catalyst containing an amine salt of sulfonic acid or a phosphine salt of sulfonic acid in production of the hydroxycarboxylic acid copolymer by dehydration polycondensation of the two or more hydroxycarboxylic acids.

4. A method of producing a polyhydroxycarboxylic acid copolymer, comprising using an organic-acid-based catalyst containing an amine salt of sulfonic acid or a phosphine salt of sulfonic acid in production of the hydroxycarboxylic acid copolymer by ring-opening-dehydration polycondensation of a hydroxycarboxylic acid and a lactone.

5. The method of producing poly-L-lactic acid according to claim 1, wherein the sulfonic acid is a fluorine-substituted alkanesulfonic acid and the phosphine is triarylphosphine.

6. The method of producing poly-L-lactic acid according to claim 5, wherein the fluorine-substituted alkanesulfonic acid is a trifluoromethanesulfonic acid and the triarylphosphine is triphenylphosphine.

## Patentansprüche

1. Verfahren zum Herstellen von Poly-L-Milchsäure, umfassend das Verwenden eines auf organischer Säure basierenden Katalysators, der ein Aminsalz von Sulfonsäure oder ein Phosphinsalz von Sulfonsäure enthält, zur Herstellung der Poly-L-Milchsäure durch Dehydratations-Polykondensation von L-Milchsäure.

2. Verfahren zum Herstellen einer Polyhydroxycarbonsäure, umfassend das Verwenden eines auf organischer Säure basierenden Katalysators, der ein Aminsalz von Sulfonsäure oder ein Phosphinsalz von Sulfonsäure enthält, zur Herstellung der Polyhydroxycarbonsäure durch Dehydratations-Polykondensation einer Hydroxycarbonsäure.

3. Verfahren zum Herstellen eines Polyhydroxycarbonsäure-Copolymers, umfassend das Verwenden eines auf organischer Säure basierenden Katalysators, der ein Aminsalz von Sulfonsäure oder ein Phosphinsalz von Sulfonsäure enthält, zur Herstellung des Hydroxycarbonsäure-Copolymers durch Dehydratations-Polykondensation der zwei oder mehr Hydroxycarbonsäuren.

4. Verfahren zum Herstellen eines Polyhydroxycarbonsäure-Copolymers, umfassend das Verwenden eines auf organischer Säure basierenden Katalysators, der ein Aminsalz von Sulfonsäure oder ein Phosphinsalz von Sulfonsäure enthält, zur Herstellung des Polyhydroxycarbonsäure-Copolymers durch Ringöffnungs-Dehydratisierungs/Polykondensation von einer Hydroxycarbonsäure und einem Lacton.

5. Verfahren zum Herstellen von Poly-L-Milchsäure gemäß Anspruch 1, wobei die Sulfonsäure eine Fluor-substituierte Alkansulfonsäure ist und das Phosphin Triarylphosphin ist.

6. Verfahren zum Herstellen von Poly-L-Milchsäure gemäß Anspruch 5, wobei die Fluor-substituierte Alkansulfonsäure eine Trifluormethansulfonsäure ist und das Triarylphosphin Triphenylphosphin ist.

## Revendications

1. Procédé de production d'acide L-polylactique, comprenant l'utilisation d'un catalyseur à base d'acide organique contenant un sel d'amine d'acide sulfonique ou un sel de phosphine d'acide sulfonique lors de la production de l'acide L-polylactique par polycondensation par déshydratation d'acide L-lactique.

2. Procédé de production d'un acide polyhydroxycarboxylique, comprenant l'utilisation d'un catalyseur à base d'acide organique contenant un sel d'amine d'acide sulfonique ou un sel de phosphine d'acide sulfonique lors de la production d'acide polyhydroxycarboxylique par polycondensation par déshydratation d'un acide hydroxycarboxylique.

3. Procédé de production d'un copolymère d'acide polyhydroxycarboxylique, comprenant l'utilisation d'un catalyseur à base d'acide organique contenant un sel d'amine d'acide sulfonique ou un sel de phosphine d'acide sulfonique lors de la production du copolymère d'acide hydroxycarboxylique par polycondensation par déshydratation des deux ou plusieurs acides hydroxycarboxyliques.

4. Procédé de production d'un copolymère d'acide polyhydroxycarboxylique, comprenant l'utilisation d'un catalyseur à base d'acide organique contenant un sel d'amine d'acide sulfonique ou un sel de phosphine d'acide sulfonique lors de la production du copolymère d'acide hydroxycarboxylique par polycondensation par déshydratation à ouverture de cycle d'un acide hydroxycarboxylique et d'une lactone.

5. Procédé de production d'acide L-polylactique selon la revendication 1, dans lequel l'acide sulfonique est un acide alcanesulfonique à fluor substitué et la phosphine est de la triarylphosphine.

6. Procédé de production d'acide L-polylactique selon la revendication 5, dans lequel l'acide alcanesulfonique à fluor substitué est un acide trifluorométhanesulfonique et la triarylphosphine est de la triphénylphosphine.
